# EUROPEAN PATENT APPLICATION

(11) **EP 4 796 644 A1**
(43) Date of publication of application: **26.08.2026**
(21) Application number: 25159480.0
(22) Date of filing: 21.02.2025
(51) Int. Cl.: C12Q 1/6804

(54) **QUANTATIVE ANTIBODY-BASED PROXIMITY ASSAY**

(71) Applicant: MU Bioteknik Aktiebolag, 181 90 Lidingö (SE)
(72) Inventor: UHLÉN, Mathias, 181 90 Lidingö (SE)
(74) Representative: Kransell & Wennborg KB

(57) **Abstract**

There is provided an assay method for determining an absolute concentration of at least one target analyte in a sample. The assay method uses a labelled target analyte and proximity-based oligonucleotide coupling to determine the absolute concentration of a target analyte in a sample.

## Description

### TECHNICAL FIELD

The present disclosure relates to the field of molecular diagnostics, specifically assays for determining the concentrations of target analytes.

### BACKGROUND

The accurate quantification of analytes such as proteins in biological samples is a cornerstone of modern molecular diagnostics and biomedical research. Traditional methods, such as enzyme-linked immunosorbent assays (ELISA), have long been employed for protein detection. While reliable, these methods have limitations in sensitivity, specificity and scalability. These shortcomings are especially problematic in applications requiring precise quantification of low-abundance analytes or analytes that require absolute concentration measurements for clinical or research purposes.

In recent years, proximity-based assays, such as proximity-based ligation assays (PLA) and proximity extension assays (PEA), have gained attention due to their ability to combine high sensitivity with multiplexing capabilities. However, there is a need for a robust, cost-effective, and highly specific assay method capable of delivering absolute quantification of target analytes in complex biological samples.

### SUMMARY

The present inventor hypothesized that by utilizing a proximity-based assay together with an internal standard, a robust and highly specific assay can be obtain wherein the absolute quantification of an analyte may be determined.

According to a first aspect, an assay method for determining an absolute concentration of a target analyte in a sample comprising:
(1) adding a labelled target analyte comprising a label in a known amount to the sample;
(2) mixing the sample comprising the target analyte and the labelled target analyte with a first binding moiety and a second binding moiety, wherein:
   (i) the first binding moiety binds specifically to the target analyte and the labelled target analyte, and comprises a first nucleic acid target label comprising a first oligonucleotide sequence;
   (ii) the second binding moiety binds specifically to the target analyte and the labelled target analyte, and comprises a second nucleic acid target label comprising a second oligonucleotide sequence;
   (iii) the first binding moiety and the second binding moiety bind to the target analyte and the labelled target analyte to form a target analyte immunocomplex and a labelled target analyte immunocomplex;
(3) optionally purifying the target analyte immunocomplex and the labelled target analyte immunocomplex;
(4) performing proximity-based oligonucleotide coupling of the first oligonucleotide sequence and the second oligonucleotide sequence of the target analyte immunocomplex and of the labelled target analyte immunocomplex to obtain a coupled target analyte immunocomplex and a coupled labelled target analyte immunocomplex;
(5) dividing the sample comprising the coupled target analyte immunocomplex and the coupled labelled target analyte immunocomplex into a first and a second subsample having a known volumetric ratio;
(6) removing the coupled labelled target analyte immunocomplex from the first subsample using the label of the coupled labelled target analyte immunocomplex;
(7) performing DNA extension of the coupled target analyte immunocomplex in the first subsample using a first PCR primer having a first sequence identity specific to the first subsample, thereby providing a first DNA extended product;
(8) performing DNA extension of the coupled target analyte immunocomplex and the coupled labelled target analyte immunocomplex in the second subsample using a second PCR primer having a second sequence identity specific to the second subsample, thereby providing a second DNA extended product;
(9) amplifying the first and the second DNA extended product to obtain a first and a second amplified product, wherein said amplification is preferably carried out after combining the first and the second DNA extended product;
(10) determining a ratio between the first and the second amplified product using the first and the second sequence identity; and
(11) determining the absolute concentration of the target analyte in the sample based on the ratio between the first and the second amplified product, the known volumetric ratio between the first and the second subsample and the known amount of the labelled target analyte added in step (1).

The use of a labelled target analyte, i.e., an internal standard, in the assay method according to the first aspect, enables the determination of the absolute concentration of a target analyte in a sample, such as a biological sample. Furthermore, the assay method enables cross-laboratory comparisons due to the use of the internal standard, and thus ensures consistency and reproducibility.

The present assay method utilizes the ratio between the target analyte and the labelled target analyte to determine the absolute concentration and thus requires less extensive sequencing. The assay method also ensures very high specificity, yielding low coefficients of variation (CV) and reliable concentration determination of low-abundance target analytes.

The method assay according to the first aspect can be used to determine the absolute concentration of multiple target analytes in a sample. The method assay can be used to determine the absolute concentration of at least two target analytes, such as at least three target analytes. Thus, the method assay may be used in multiplexing.

According to a second aspect, there is provided a kit for determining an absolute concentration of a target analyte in a sample comprising:
- a first binder comprising a first nucleic acid target label having a first oligonucleotide sequence;
- a second binder comprising a second nucleic acid target label having a second oligonucleotide sequence;
- a first nucleic acid coupling agent; and
- a labelled target analyte comprising a label wherein:
   (i) the first and second binder specifically bind to the target analyte and the labelled target analyte; and
   (ii) the first and second oligonucleotide sequence interacts with the nucleic acid coupling agent.

The kit according to the second aspect enables the determination of the absolute concentration of the target analyte with high specificity and low CV. The kit further enables reliable determination of the absolute concentration of low-abundance target analytes.

### BRIEF DESCRIPTION OF THE DRAWINGS

Fig 1 shows a schematic view of a binding moiety according to the present disclosure.

Fig 2 shows the product of proximity-based ligation wherein the pentagon represents the target analyte and the pentagon with "B" represents the labelled target analyte.

Fig 3 shows an example of the structure of a first and a second binding moiety. T1 - first oligonucleotide sequence, S ID - sequence identity specific to the sample, , SS ID - a sequence identity specific to the subsample, P1- a PCR primer, P3 - a sequence primer, H - general sequence. T2 - first oligonucleotide sequence, P2- a PCR primer.

Fig 4 shows illustrative examples of the calculation of the ratio between the first amplified product (first subsample) and the second amplified product (second subsample) using the first and the second sequence identity. The pentagon represents the target analyte and the pentagon with "B" represents the labelled target analyte.

Figure 5 shows a simplified overview of the assay method of the first aspect. The pentagon represents the target analyte and the pentagon with "B" represents the labelled target analyte.

### DETAILED DESCRIPTION

The proximity-based assays available at the present are readily used for diagnostic purposes. However, these assays often lack robustness, high specificity and the ability to determine absolute concentrations of a target analyte. The present disclosure uses a labelled target analyte, i.e., an internal standard, to induce robustness and control to the assay while enabling the determination of the absolute concentration of a target analyte in a biological sample.

According to a first aspect, an assay method for determining an absolute concentration of a target analyte in a sample comprising:
(1) adding a labelled target analyte comprising a label in a known amount to the sample;
(2) mixing the sample comprising the target analyte and the labelled target analyte with a first binding moiety and a second binding moiety, wherein:
   (i) the first binding moiety binds specifically to the target analyte and the labelled target analyte, and comprises a first nucleic acid target label comprising a first oligonucleotide sequence;
   (ii) the second binding moiety binds specifically to the target analyte and the labelled target analyte, and comprises a second nucleic acid target label comprising a second oligonucleotide sequence;
   (iii) the first binding moiety and the second binding moiety bind to the target analyte and the labelled target analyte to form a target analyte immunocomplex and a labelled target analyte immunocomplex;
(3) optionally purifying the target analyte immunocomplex and the labelled target analyte immunocomplex;
(4) performing proximity-based oligonucleotide coupling of the first oligonucleotide sequence and the second oligonucleotide sequence of the target analyte immunocomplex and of the labelled target analyte immunocomplex to obtain a coupled target analyte immunocomplex and a coupled labelled target analyte immunocomplex;
(5) dividing the sample comprising the coupled target analyte immunocomplex and the coupled labelled target analyte immunocomplex into a first and a second subsample having a known volumetric ratio;
(6) removing the coupled labelled target analyte immunocomplex from the first subsample using the label of the coupled labelled target analyte immunocomplex;
(7) performing DNA extension of the coupled target analyte immunocomplex in the first subsample using a first PCR primer having a first sequence identity specific to the first subsample, thereby providing a first DNA extended product;
(8) performing DNA extension of the coupled target analyte immunocomplex and the coupled labelled target analyte immunocomplex in the second subsample using a second PCR primer having a second sequence identity specific to the second subsample, thereby providing a second DNA extended product;
(9) amplifying the first and the second DNA extended product to obtain a first and a second amplified product, wherein said amplification is preferably carried out after combining the first and the second DNA extended product;
(10) determining a ratio between the first and the second amplified product using the first and the second sequence identity; and
(11) determining the absolute concentration of the target analyte in the sample based on the ratio between the first and the second amplified product, the known volumetric ratio between the first and the second subsample and the known amount of the labelled target analyte added in step (1).

The use of a labelled target analyte in the assay method of the present disclosure enables the determination of the absolute concentration of a target analyte in a biological sample. Furthermore, the assay method enables cross-laboratory comparisons due to the use of an internal standard, i.e., the labelled target analyte, and thus ensures consistency and reproducibility.

The assay method further uses the ratio between the target analyte and the labelled target analyte to determine the absolute concentration and thus requires less extensive sequencing. The assay method according to the first aspect ensures very high specificity, yielding low CV and reliable concentration determination of low-abundance target analytes.

The assay method according to the first aspect may be used to determine the absolute concentration of multiple target analytes in a sample, so called multiplexing. Preferably, the assay method is used to determine the absolute concentration of at least two target analytes, such as at least three target analytes. The assay method of the first aspect may be used to determine the absolute concentration of thousands of target analytes simultaneously.

In one embodiment, the assay method comprises determining the absolute concentration of a second target analyte in the sample. The assay method thus comprises:
(1a) adding a labelled second target analyte comprising a label in a known amount to the sample;
(2a) mixing the sample comprising the second target analyte and the labelled second target analyte with a third binding moiety and a fourth binding moiety, wherein:
   (iv) the third binding moiety binds specifically to the second target analyte and the labelled second target analyte, and comprises a third nucleic acid target label comprising a third oligonucleotide sequence;
   (v) the fourth binding moiety binds specifically to the second target analyte and the labelled second target analyte, and comprises a fourth nucleic acid target label comprising a fourth oligonucleotide sequence;
   (vi) the third binding moiety and the fourth binding moiety bind to the second target analyte and the labelled second target analyte to form a second target analyte immunocomplex and a labelled second target analyte immunocomplex;
(3a) optionally purifying the second target analyte immunocomplex and the labelled second target analyte immunocomplex;
(4a) performing proximity-based oligonucleotide coupling between the third oligonucleotide sequence and the fourth oligonucleotide sequence of the second target analyte immunocomplex and of the labelled second target analyte immunocomplex to obtain a coupled second target analyte immunocomplex and a coupled labelled second target analyte immunocomplex;
(5a) dividing the sample comprising the coupled target analyte immunocomplex and the coupled labelled target analyte immunocomplex into the first and the second subsample having a known volumetric ratio;
(6a) removing the coupled labelled second target analyte immunocomplex from the first subsample using the label of the coupled labelled second target analyte immunocomplex;
(7a) performing DNA extension of the coupled second target analyte immunocomplex in the first subsample using a third PCR primer having a third sequence identity specific to the first subsample, thereby providing a third DNA extended product;
(8a) performing DNA extension of the coupled second target analyte immunocomplex and the coupled labelled second target analyte immunocomplex in the second subsample using a fourth PCR primer having a fourth sequence identity specific to the second subsample, thereby providing a fourth DNA extended product;
(9a) amplifying the third and the fourth DNA extended product to obtain a third and a fourth amplified product, wherein said amplification is preferably carried out after combining the third and the fourth DNA extended product;
(10a) determining a ratio between the third and the fourth amplified product using the third and the fourth sequence identity; and

(11a) determining the absolute concentration of the second target analyte in the sample based on the ratio between the third and the fourth amplified product, the known volumetric ratio between the first and the second subsample and the known amount of the labelled second target analyte added in step (1a).

The steps 1a-11a, respectively, are performed simultaneously as steps 1-11, respectively.

The assay method may further comprise determining the absolute concentration of at least a third target analyte in the sample. Preferably, the assay method comprises determining the absolute concentration of multiple target analytes such as up to thousands of target analytes. Steps 1-11 in the assay method may be performed for each target analyte that an absolute concentration is to be determined. For each target analyte a specific labelled target analyte and binding moieties that specifically bind to the target analytes are added.

For each specific target analyte that an absolute concentration is to be determined, there is added a specific labelled target analyte and binding moieties that bind specifically to said target analyte.

All embodiments disclosed below relating to the determination of the absolute concentration of a target analyte apply *mutatis mutandis* to all target analytes in a sample for which an absolute concentration is to be determined.

The target analyte may be any substance or entity for which an absolute concentration needs to be determined in a sample. The target analyte may be a proteinaceous molecule such as a polypeptide, protein or prion. The target molecule may be any molecule which comprises a protein, a polypeptide component, or a fragment thereof. The target analyte may be a single molecule or a complex that contains two or more molecular subunits, which may or may not be covalently bound to one another, and which may be the same or different. Some examples of target analytes are Interleukin 10, Interleukin-17A, TNF-α and alpha fetoprotein.

The sample may be any biological or clinical samples comprising the target analyte. For example, the sample may be any cell or tissue sample of an organism, preferably a human. In one embodiment, the sample may be of a body fluid, such as blood, or a preparation derived thereof. Preferably, the sample is a blood sample. The sample may be freshly prepared or pre-treated in any conventional way.

The labelled target analyte is a target analyte that has been modified with a label prior to use in the assay method according to the present disclosure. The label may any functional group capable of binding to a capture molecule with high affinity, such as antibodies and biotin. Preferably, the labelled target analyte is a biotinylated target analyte. The biotin may be bound to the target analyte covalently through any biotinylation reaction commonly known in art. A skilled person in the art would know which biotinylation reaction to choose depending on the target analyte that is to be biotinylated. The label of the labelled target analyte allows for specific capture of the labelled target analyte.

The labelled target analyte is used as an internal standard in the assay method according to the first aspect and enables an easy way of determining the absolute concentration of the target analyte in the sample. The determination of the absolute concentration in this disclosure is based on the ratio of the target analyte and the labelled target analyte and thus requires less sequencing. Furthermore, the labelled target analyte enables cross-laboratory comparison and thus ensures consistency and reproducibility of the assay method.

For each target analyte for which an absolute concentration is to be determined, a specific labelled target analyte is added to the sample.

The labelled target analyte is added to the sample in a known amount. Adding the labelled target analyte in a known amount enables the determination the absolute concentration of the target analyte in the sample as the absolute concentration is determined using the ratio of the target analyte and the labelled target analyte and the known amount of the labelled target analyte. The labelled target analyte is preferably added in an amount such that a similar concentration is obtained in the sample as the "normal concentrations" of the target analyte in the sample. "Normal concentration" refers to the concentration in which the target analyte is generally found in the sample. For example, the protein Interleukin-17a is typically present in the blood in a concentration of 0.2-20 ng/L. Thus, if Interleukin-17a is the target analyte, the labelled target analyte, i.e., labelled Interleukin-17a, would be added to the sample in such an amount that the concentration of the labelled Interleukin-17a in the sample is in the same range as 0.2-20 ng/L.

The dynamic range of the method assay may be altered by altering the amount of added labelled target analyte.

The assay method relies on the principles of "proximity probing", wherein a target analyte is detected by the binding of multiple probes, i.e. two or more probes, which when brought into proximity by binding to the target analyte allow a signal to be generated. The signal generation (used for detection) is dependent on an interaction between the probes and hence only occurs when both the necessary probes have bound to the target analyte. In the present disclosure the probes are the binding moieties that bind specifically to the target analyte and to the labelled target analyte. When determining the absolute concentration of multiple target analytes, binding moieties that specifically bind to each target analyte are added to the sample.

All embodiments disclosed below relating to the determination of the absolute concentration of a target analyte may be applied to all target analytes in a sample for which an absolute concentration is to be determined.

A sample comprising a target analyte is provided. A known amount of a labelled target analyte is added to the sample and the sample is preferably mixed. The sample comprising the target analyte and the labelled target analyte is mixed with a first binding moiety and a second binding moiety.

The first and the second binding moiety may each comprise an antibody, an antibody fragment, an affibody, a SOMAmer or an aptamer.

The first binding moiety binds specifically to a first site on the target analyte and to a first site on the labelled target analyte. The first binding moiety binds to the same site on both the target analyte and the labelled analyte and thus binds to both the target analyte and the labelled target analyte equally.

The second binding moiety binds specifically to a second site on the target analyte and on the labelled target analyte. The second binding moiety binds to the same site on both the target analyte and the labelled analyte and thus binds to both the target analyte and the biotinylated target analyte equally.

The first and second site are non-interfering sites present on both the target analyte and the labelled target analyte.

It is to be understood in the present disclosure that one first binding moiety, i.e., binding molecule, binds to the target analyte and another first binding moiety, i.e., binding molecule, binds to the labelled target analyte. Thus, one first binding moiety does not bind to both the target analyte and the labelled target analyte. The same is applicable for the second binding moiety.

The term "bind" refers to the interaction of the binding moieties with the sites on the target analyte and on the labelled target analyte, and the formation of complexes. The complexes are thus formed through the interaction of the binding moieties and the target analyte or through the interaction of the binding moieties and the labelled target analyte. The interactions may be non-covalent such as ionic bonding, hydrogen bonding, Van der Waals interactions, hydrophobic interactions or combinations thereof.

The first and the second binding moiety binds specifically to a first or second site on the target analyte and the labelled target analyte. This means that the first and second binding moiety have a greater affinity to the target analyte and to the labelled target analyte than to other compounds in the sample. For example, the first binding moiety and the second binding moiety binding to the target analyte and to the labelled target analyte can be distinguished from their binding to non-target analytes present in the sample in that the first and second binding moiety either does not bind to non-target analytes or does so negligibly or non-detectably. If any such non-specific binding would occur during the assay method, it would occur at a relatively low level that is clearly distinguishable from the binding to the target analyte and to the labelled target analyte.

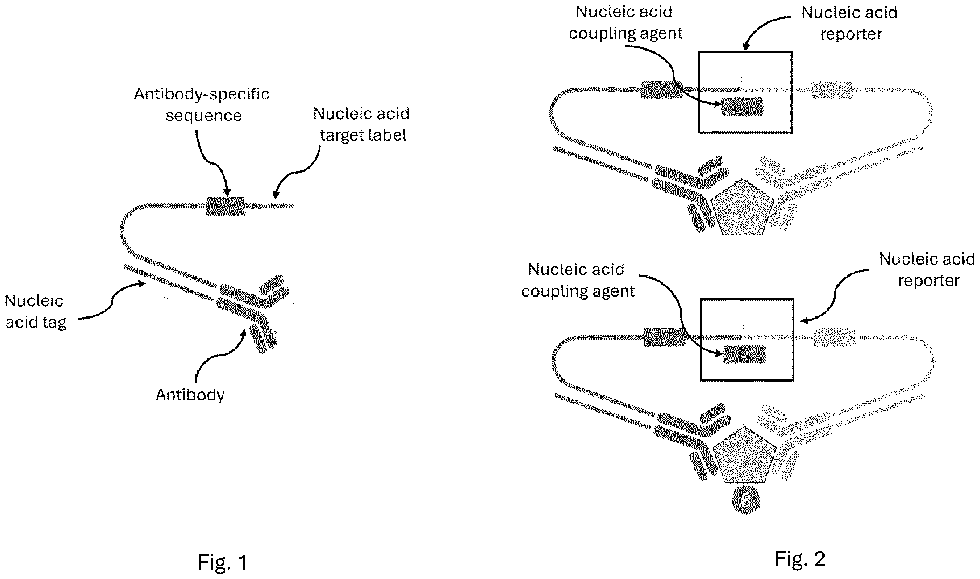

In a preferred embodiment, the first binding moiety comprises a first antibody or a first antibody fragment and the second binding moiety comprises a second antibody or a second antibody fragment. The first and the second binding moieties comprising antibodies or antibody fragments are schematically depicted in Figure 1. The first antibody or the first antibody fragments and the second antibody or the second antibody fragments binds specifically to the target analyte and the labelled target analyte. The first and the second antibody may be monoclonal or polyclonal.

The first and second antibody fragments preferably maintain the binding characteristics of the parent antibodies.

The first binding moiety and the second binding moiety bind to the target analyte and to the labelled target analyte to form a target analyte immunocomplex and a labelled target analyte immunocomplex. Thus, the first binding moiety binds to a first site on the target analyte and on the labelled target analyte while the second binding moiety binds to a second site on the target analyte and on the labelled target analyte. The target analyte immunocomplex and the labelled immunocomplex are both present in the sample after the addition of the first and second binding moiety. When the first binding moiety and the second binding moiety bind to the target analyte and to the labelled target analyte, the first and the second binding moiety are positioned in close proximity to each other and thus enabling proximity-based oligonucleotide coupling at a later stage.

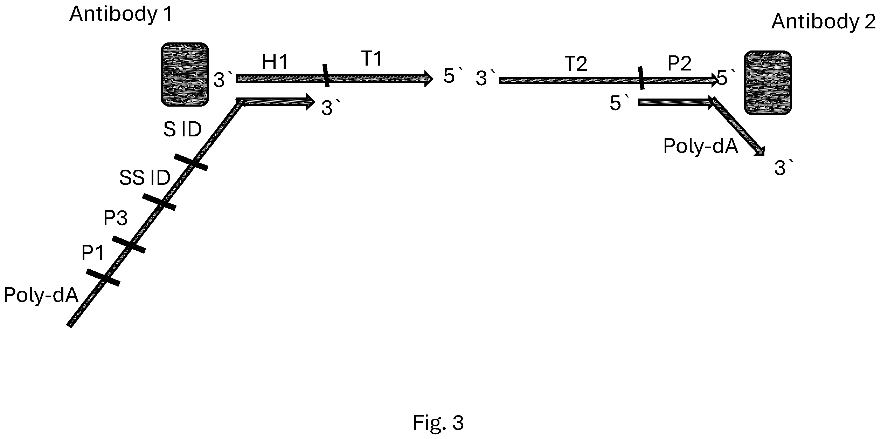

The first binding moiety further comprises a first nucleic acid target label comprising a first oligonucleotide sequence and the second binding moiety comprises a second nucleic acid target label comprising a second oligonucleotide sequence, see Figure 1. The first oligonucleotide sequence of the first binding moiety may be coupled to the first antibody or the first antibody fragment by its 5' end while the second oligonucleotide sequence of the second binding moiety is coupled to the second antibody or the second antibody fragment by its 3' end, see Figure 3. Thus, when brought into proximity to each other via the binding to the target analyte or the labelled target analyte, the free 3' hydroxyl of the first oligonucleotide sequence will be capable of reacting with the 5' phosphate of the second oligonucleotide sequence.

The first binding moiety may further comprise a first nucleic acid tag comprising an oligonucleotide sequence, such as a poly-dA sequence or a poly-dG sequence, see Figure 1 and 3. Preferably, the oligonucleotide sequence of the first nucleic acid tag is a poly-dA sequence.

The second binding moiety may also comprise a second nucleic acid tag comprising an oligonucleotide sequence, such as a poly-dA sequence or a poly-dG sequence, see Figure 1 and 3. Preferably, the oligonucleotide sequence of the second nucleic acid tag is a poly-dA sequence.

Preferably, both the first and the second binding moiety comprises a first and second nucleic tag and wherein the oligonucleotide sequence of both the first and the second nucleic acid tags comprises poly-dA sequences.

The first binding moiety may further comprise a first antibody specific sequence, see Figure 1. Likewise, the second binding moiety may comprise a second antibody specific sequence, see Figure 1.

In a preferred embodiment, the target analyte immunocomplex and the labelled target analyte immunocomplex are purified. Preferably, the purification is performed using a capture-release mechanism. The purification of the immunocomplexes may reduce the non-specific background signals of the sample during detection and thus improve the detection of the target analyte.

The purification of the analyte immunocomplex and the labelled immunocomplex may be obtained by introducing first paramagnetic beads and capturing the target analyte immunocomplex and the labelled target analyte immunocomplex on said first paramagnetic beads. The first paramagnetic beads may comprise oligonucleotide sequences that are complementary to the oligonucleotide sequences in the first and/or second nucleic acid tags, preferably the first paramagnetic beads comprise a poly-dT sequence or a poly-dC sequence.

The target analyte immunocomplex and of the labelled target analyte immunocomplex may be captured by hybridization of the first and/or second nucleic acid tag with the oligonucleotide sequences of the first paramagnetic beads.

Preferably, the first and/or second nucleic acid tag comprises a poly-dA sequence and the first paramagnetic beads comprises a poly-dT sequence, and the capturing of the analyte immunocomplex and the labelled target analyte immunocomplex occurs by hybridization between the poly-dA sequence of the first and/or second nucleic acid tag and the poly-dT sequence of the first paramagnetic beads.

The purification may further comprise washing the first paramagnetic beads with the captured target analyte immunocomplex and the captured labelled target analyte immunocomplex. The paramagnetic beads are preferably washed with an aqueous solution. The washing may remove any unbound molecules present.

The purification may also comprise releasing the target analyte immunocomplex and labelled target analyte immunocomplex from the first paramagnetic beads. The releasing may occur by disrupting the hybridization between the oligonucleotide sequences of the paramagnetic beads and the first and/or second nucleic acid tags. However, the target analyte immunocomplex and the labelled target analyte immunocomplex does not need to be released from the first paramagnetic beads prior to the proximity-based oligonucleotide coupling.

After the optional purification of the target analyte immunocomplex and the labelled target analyte immunocomplex, a proximity-based oligonucleotide coupling of the first oligonucleotide sequence and the second oligonucleotide sequence of the target analyte immunocomplex and of the labelled target analyte immunocomplex is performed to obtain a coupled target analyte immunocomplex and a coupled labelled target analyte immunocomplex.

The proximity-based oligonucleotide coupling is preferably proximity-based ligation or proximity-based extension, more preferably it is proximity-based ligation.

To enable the proximity-based oligonucleotide coupling, preferably a nucleic acid coupling agent is added.

When the proximity-based oligonucleotide coupling is proximity-based ligation, step 4/4a comprises:
- adding a single-stranded nucleic acid coupling agent to the sample comprising the target analyte immunocomplex and the labelled target analyte immunocomplex wherein the single-stranded nucleic acid coupling agent comprises a first segment hybridizing the first nucleic acid target label, and a second segment hybridizing the second nucleic acid target label, and completing proximity-based ligation, following hybridization between the single-stranded nucleic acid coupling agent and the first and the second oligonucleotides of the first and the second nucleic acid target labels
   in the target analyte immunocomplex and in the labelled target analyte immunocomplex, respectively, to obtain a coupled target analyte immunocomplex and a coupled labelled target analyte immunocomplex wherein a first nucleic acid reporter bridges the first and the second oligonucleotides in the target analyte immunocomplex and a second nucleic acid reporter bridges the first and the second oligonucleotides in the labelled target analyte immunocomplex, respectively, see Figure 2.

The single-stranded nucleic acid coupling agent thus has a first end comprising an oligonucleotide sequence complementary to the first oligonucleotide sequence of the first nucleic acid target label and a second end comprising an oligonucleotide sequence complementary to the second oligonucleotide sequence of the second nucleic acid target label.

The proximity-based ligation means that the first oligonucleotide sequence of the first nucleic acid target label and the second oligonucleotide sequence of the second nucleic acid target label interact by ligation to the oligonucleotide sequence of the single-stranded nucleic acid coupling agent. The signal generation when detecting the target analyte is thus dependent on the product obtained after completing the proximity-based ligation.

When the proximity-based oligonucleotide coupling is proximity-based extension, step 4/4a comprises:
- forming a duplex wherein the first oligonucleotide of the first nucleic acid target label and the second oligonucleotide of the second nucleic acid target label interact with each other, and extending the at least first oligonucleotide and/or the second oligonucleotide of said duplex to obtain a coupled target analyte immunocomplex and a coupled labelled target analyte immunocomplex.

The extension of the proximity-based extension may be performed using a DNA polymerase.

The sample comprising the coupled target analyte immunocomplex and the coupled labelled target analyte immunocomplex may be washed and/or eluted with salt after the completion of the proximity-based oligonucleotide coupling. The washing may remove ligation or extension products other than the coupled target analyte immunocomplex and the coupled labelled target analyte immunocomplex. After the elution, the supernatant may be selected and divided into a first and a second subsample having a known volumetric ratio. However, the step of washing and/or eluting with salt is not necessary. Thus, the sample comprising the coupled target analyte immunocomplex and the coupled labelled target analyte immunocomplex may be divided into a first and a second subsample having a known volumetric ratio directly after completing the proximity-based oligonucleotide coupling.

The next step in the assay method according to the first aspect is the removal of the coupled labelled target analyte immunocomplex from the first subsample using the label of coupled labelled target analyte immunocomplex. The coupled labelled target analyte immunocomplex is thus completely removed from the first subsample.

The coupled labelled target analyte immunocomplex in step (6) may be removed using a second paramagnetic beads. Preferably, the second paramagnetic beads are modified such that they can interact with the label of the coupled labelled target analyte immunocomplex.

If the labelled target analyte is biotinylated target analyte, the second paramagnetic beads are preferably modified with streptavidin. The biotin of the coupled biotinylated target analyte immunocomplex may thus interact with the streptavidin of the second paramagnetic beads to form a streptavidin-biotin complex. Thus, by removing the second paramagnetic beads, the coupled biotinylated target analyte immunocomplex is also removed.

The second paramagnetic beads may be removed using a magnet.

DNA extension of the coupled target analyte immunocomplex in the first subsample is performed. The DNA extension is performed using a first PCR primer having a first sequence identity specific to the first subsample, thereby providing a first DNA extended product. The first PCR primer is preferably complementary to the first and second oligonucleotide sequence of the coupled target analyte immunocomplex. The DNA extension step ensures that the DNA templates are complete and double-stranded, that they are ready for down-stream amplification and that the first sequence identity is incorporated.

DNA extension of the coupled target analyte immunocomplex and the coupled labelled target analyte immunocomplex in the second subsample is performed. The DNA extension is performed using a second PCR primer having a second sequence identity specific to the second subsample, thereby providing a second DNA extended product. The second PCR primer is preferably complementary to the first and the second oligonucleotide sequence of the coupled target analyte immunocomplex and the coupled labelled target analyte immunocomplex.

During the DNA extension, a sample specific nucleotide sequence and a sequencing primer may be added to the first or the second nucleic acid target label.

The DNA extension in step (7) and (8) may be performed using thermostable polymerase such as Taq polymerase.

The first and the second DNA extended product are amplified to obtain a first and a second amplified product. The amplification is preferably carried out after combining the first and the second DNA extended product. Combining the first and the second DNA extended product prior to amplification enables more control over the reactions and more homogeneous results. The amplification is preferably obtained by performing PCR.

The first and the second DNA extended products may be purified prior to amplification using a third paramagnetic breads. The first and the second DNA extended product are captured using the third paramagnetic beads and may be washed to remove any unbound molecules. Preferably, the first and the second DNA extended products are released from the third paramagnetic beads prior to detection.

Sequencing is preferably performed on the first and the second amplified product. Preferably, the sequencing is obtained by Next Generation Sequencing (NGS).

A ratio between the first and the second amplified product is determined using the ratio of the first and the second sequence identity. The ratio is based on read count from the sequencing.

The absolute concentration of the target analyte in the sample is determined based on the ratio between the first and the second amplified product, the known volumetric ratio between the first and the second subsample and the known amount of the labelled target analyte added in step (1).

Figure 3 shows an example of the structure of a first and a second binding moiety prior to amplification. The first binding moiety comprises Antibody 1 that specifically binds to the target analyte and the labelled target analyte. The first binding moiety further comprises a first nucleic acid target label comprising a first oligonucleotide sequence, T1. The first binding moiety further comprises a general sequence, H1, that allows annealing of the sequences produced during DNA extension. PCR primer, P1, is used during the DNA extension. During the DNA extension a sequence identity specific to the sample, S ID, a sequence identity specific to the subsample, SS ID and a sequence primer, P3, are introduced. The first binding moiety of Figure 3 further comprises a first nucleic acid tag comprising a poly-dA sequence.

The second binding moiety shown in Figure 3, comprises Antibody 2 that specifically binds to the target analyte and the labelled target analyte. The second binding moiety further comprises a second nucleic acid target label comprising a second oligonucleotide sequence, T2. The second binding moiety of Figure 3 further comprises a second nucleic acid tag comprising a poly-dA sequence and a PCR primer, P2.

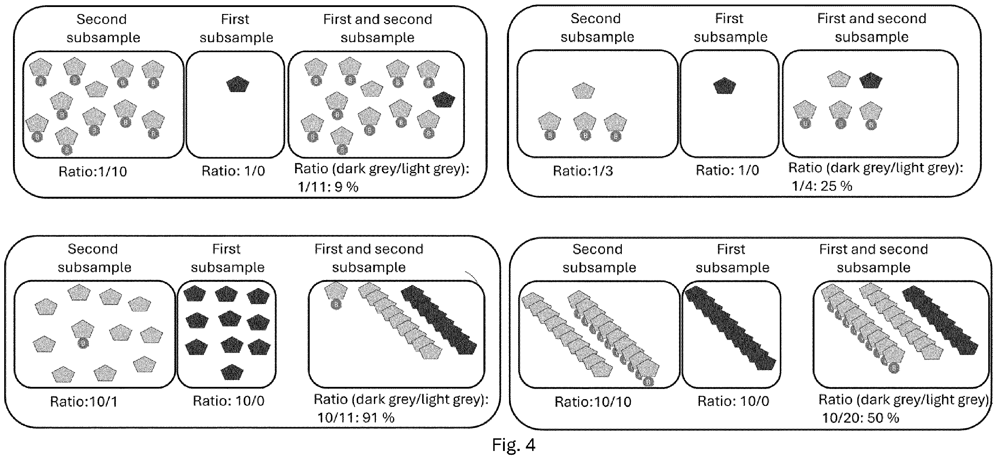

Figure 4 show four illustrative examples of the calculation of the ratio between the first and the second amplified product using the first and the second sequence identity. Table 1 shows other examples of calculating the ratio between the first and second amplified product using the first and second sequence identity.

**Table 1 shows the calculation of the ratio between the first amplified and the second amplified product and thus the ratio between the target analyte and the labelled target analyte. "reads" corresponds to XX.**

| Target analyte (amount in sample) | Labelled target analyte (added amount) | First Subsample (reads) | Second subsample (reads) | Ratio target analyte/ labelled target analyte |
|---|---|---|---|---|
| 1 | 100 | 1 | 101 | 1% |
| 10 | 100 | 10 | 110 | 9% |
| 100 | 100 | 100 | 200 | 50% |
| 1000 | 100 | 1000 | 1100 | 91% |
| 10000 | 100 | 10000 | 10100 | 99% |

Table 1 furthermore shows the dynamic range of the assay method according to the first aspect. The dynamic range of the present assay method may be 100× up and down from "normal concentration" of the target analyte in the sample. "Normal concentration" refers to the concentration in which the target analyte is generally found in the sample.

The assay method according to the first aspect may easily be automated.

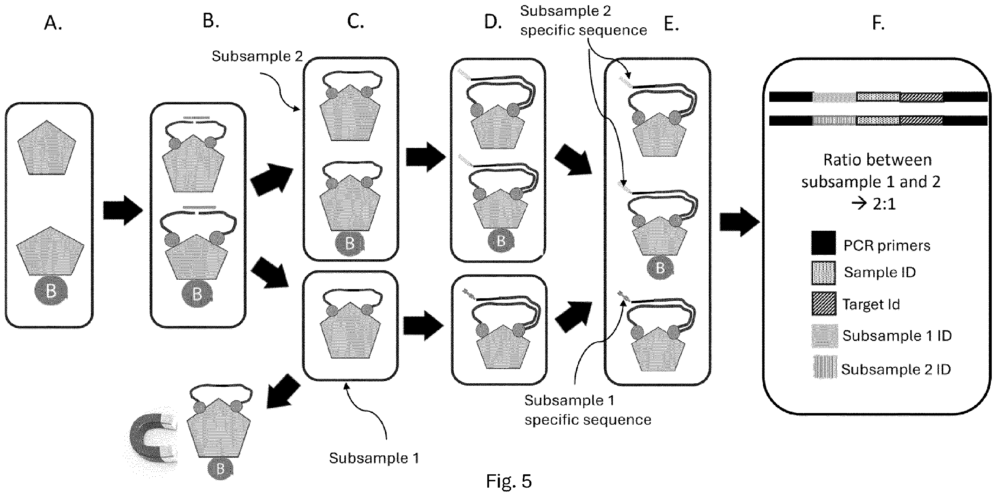

Figure 5 discloses a simplified overview of the assay method of the first aspect. A sample comprising a target analyte is provided and a biotinylated target analyte is added to the sample in A). In B) the first and second binding moiety is added to the sample and proximity-based oligonucleotide coupling is performed. This is followed by dividing the sample into two subsamples in C) and removing the coupled biotinylated target analyte from the first subsample using a magnet. In D) DNA extension is performed on both subsamples introducing sequence identity specific to each subsample. In E) the subsamples are mixed, and amplification is performed. In F) sequencing is performed and based on the ratio between the reads of the first subsample and the second subsample, an absolute concentration of the target analyte is determined.

According to a second aspect, a kit for determining an absolute concentration of a target analyte in a sample comprising:
- a first binder comprising a first nucleic acid target label having a first oligonucleotide sequence;
- a second binder comprising a second nucleic acid target label having a second oligonucleotide sequence;
- a first nucleic acid coupling agent; and
- a labelled target analyte comprising a label wherein:
   (i) the first and second binder specifically bind to the target analyte and the labelled target analyte; and
   (ii) the first and second oligonucleotide sequence interacts with the nucleic acid coupling agent.

The kit according to the second aspect may be used to perform the assay method according to the first aspect. Thus, the kit according to the second aspect enables determining the absolute concentration of the target analyte with high specificity and low CV. The kit enables reliable determination of the concentration of low-abundance target analytes.

The kit according to the second aspect may further comprise:
- a third binder comprising a third nucleic acid target label having a third oligonucleotide sequence;
- a fourth binder comprising a fourth nucleic acid target label having a fourth oligonucleotide sequence;
- a second nucleic acid coupling agent; and
- a labelled second target analyte comprising a label wherein:
   (i) the third and fourth binder specifically bind to the second target analyte and to the labelled second target analyte; and
   (ii) the third and fourth oligonucleotide sequence interacts with the second nucleic acid coupling agent.

The first and second nucleic acid coupling agent enables proximity-based oligonucleotide coupling such as proximity-based ligation and proximity-based extension.

The labelled target analyte is preferably a biotinylated target analyte.

The first binder preferably comprises an antibody, an antibody fragment, an affibody, a SOMAmer or an aptamer. More preferably, the first binder comprises a first antibody or a first antibody fragment that specifically binds to the target analyte and the labelled target analyte.

Likewise, the second binder preferably comprises an antibody, an antibody fragment, an affibody, a SOMAmer or an aptamer. More preferably, the first binder comprises a second antibody or a second antibody fragment that specifically binds to the target analyte and the labelled target analyte.

The nucleic acid coupling agent together with the first and second nucleic acid target label enable performing proximity-based oligonucleotide coupling.

The first and second nucleic acid coupling agent may be a single-stranded nucleic acid coupling agent if proximity-based ligation is to be performed. The first and second nucleic acid coupling agent may comprise a first segment hybridizing the first oligonucleotide sequence of the first nucleic acid target label, and a second segment hybridizing the second oligonucleotide sequence of the second nucleic acid target label.

The first binder may further comprise a first nucleic acid tag comprising an oligonucleotide sequence, such as a poly-dA sequence or a poly-dG sequence.

Likewise, the second binder may further comprise a second nucleic acid tag comprising an oligonucleotide sequence, such as a poly-dA sequence or a poly-dG sequence. The first and/or the second nucleic acid tag facilitates purification of the formed immunocomplexes.

The kit may further comprise at least one set of paramagnetic beads. A first set of paramagnetic beads may be modified to interact with the nucleic acid tags on the binders. The kit may further comprise a second set of paramagnetic beads that may be modified to interact with the label of the labelled target analyte.

## Claims

1. An assay method for determining an absolute concentration of a target analyte in a sample comprising:
(1) adding a labelled target analyte comprising a label in a known amount to the sample;
(2) mixing the sample comprising the target analyte and the labelled target analyte with a first binding moiety and a second binding moiety, wherein:
(i) the first binding moiety binds specifically to the target analyte and the labelled target analyte, and comprises a first nucleic acid target label comprising a first oligonucleotide sequence;
(ii) the second binding binds specifically to the target analyte and the labelled target analyte, and comprises a second nucleic acid target label comprising a second oligonucleotide sequence;
(iii) the first binding moiety and the second binding moiety bind to the target analyte and the labelled target analyte to form a target analyte immunocomplex and a labelled target analyte immunocomplex;
(3) optionally purifying the target analyte immunocomplex and the labelled target analyte immunocomplex;
(4) performing proximity-based oligonucleotide coupling of the first oligonucleotide sequence and the second oligonucleotide sequence of the target analyte immunocomplex and of the labelled target analyte immunocomplex to obtain a coupled target analyte immunocomplex and a coupled labelled target analyte immunocomplex;
(5) dividing the sample comprising the coupled target analyte immunocomplex and the coupled labelled target analyte immunocomplex into a first and a second subsample having a known volumetric ratio;
(6) removing the coupled labelled target analyte immunocomplex from the first subsample using the label of the coupled labelled target analyte immunocomplex;
(7) performing DNA extension of the coupled target analyte immunocomplex in the first subsample using a first PCR primer having a first sequence identity specific to the first subsample, thereby providing a first DNA extended product;
(8) performing DNA extension of the coupled target analyte immunocomplex and the coupled labelled target analyte immunocomplex in the second subsample using a second PCR primer having a second sequence identity specific to the second subsample, thereby providing a second DNA extended product;
(9) amplifying the first and the second DNA extended product to obtain a first and a second amplified product, wherein said amplification is preferably carried out after combining the first and the second DNA extended product;
(10) determining a ratio between the first and the second amplified product using the first and the second sequence identity; and
(11) determining the absolute concentration of the target analyte in the sample based on the ratio between the first and the second amplified product, the known volumetric ratio between the first and the second subsample and the known amount of the labelled target analyte added in step (1).

2. The assay method according to claim 1, wherein the labelled target analyte is a biotinylated target analyte.

3. The assay method according to any one of claims 1-2, wherein the first binding moiety comprises a first antibody or a first antibody fragment and the second binding moiety comprises a second antibody or a second antibody fragment.

4. The assay method according to any one of the preceding claims, wherein the proximity-based oligonucleotide coupling is proximity-based ligation or proximity-based extension, preferably proximity-based ligation.

5. The assay method according to any one of the preceding claims, wherein the amplification is obtained by performing PCR.

6. The assay method according to any one of the preceding claims, wherein sequencing is performed on the first and second amplified product, preferably Next Generation Sequencing is performed on the first and second amplified product.

7. The assay method according to any one of the preceding claims, wherein the purification of the target analyte immunocomplex and the labelled target analyte immunocomplex in step (3) is obtained by introducing a first paramagnetic beads and capturing the target analyte immunocomplex and the labelled target analyte immunocomplex on said first paramagnetic beads.

8. The assay method according to any one of the preceding claims, wherein the coupled labelled second target analyte immunocomplex in step (6) is removed from the first subsample using a second paramagnetic beads.

9. The assay method according to any one of the preceding claims, wherein the first and second DNA extended product are purified prior to amplification.

10. The assay method according to any one of the preceding claims, wherein the assay method comprises determining the absolute concentration of a second target analyte in the sample.

11. The assay method according to claim 10, further comprising:
(1a) adding a labelled second target analyte comprising a label in a known amount to the sample;
(2a) mixing the sample comprising the second target analyte and the labelled second target analyte with a third binding moiety and a fourth binding moiety, wherein:
(iv) the third binding moiety binds specifically to the second target analyte and the labelled second target analyte, and comprises a third nucleic acid target label comprising a third oligonucleotide sequence;
(v) the fourth binding moiety binds specifically to the second target analyte and the labelled second target analyte, and comprises a fourth nucleic acid target label comprising a fourth oligonucleotide sequence;
(vi) the third binding moiety and the fourth binding moiety bind to the second target analyte and the labelled second target analyte to form a second target analyte immunocomplex and a labelled second target analyte immunocomplex;
(3a) optionally purifying the second target analyte immunocomplex and the labelled second target analyte immunocomplex;
(4a) performing proximity-based oligonucleotide coupling between the third oligonucleotide sequence and the fourth oligonucleotide sequence of the second target analyte immunocomplex and of the labelled second target analyte immunocomplex to obtain a coupled second target analyte immunocomplex and a coupled labelled second target analyte immunocomplex;
(5a) dividing the sample comprising the coupled target analyte immunocomplex and the coupled labelled target analyte immunocomplex into the first and the second subsample having a known volumetric ratio;
(6a) removing the coupled labelled second target analyte immunocomplex from the first subsample using the label of the coupled labelled second target analyte immunocomplex;
(7a) performing DNA extension of the coupled second target analyte immunocomplex in the first subsample using a third PCR primer having a third sequence identity specific to the first subsample, thereby providing a third DNA extended product;
(8a) performing DNA extension of the coupled second target analyte immunocomplex and the coupled labelled second target analyte immunocomplex in the second subsample using a fourth PCR primer having a fourth sequence identity specific to the second subsample, thereby providing a fourth DNA extended product;
(9a) amplifying the third and the fourth DNA extended product to obtain a third and a fourth amplified product, wherein said amplification is preferably carried out after combining the third and the fourth DNA extended product;
(10a) determining a ratio between the third and the fourth amplified product using the third and the fourth sequence identity; and
(11a) determining the absolute concentration of the second target analyte in the sample based on the ratio between the third and the fourth amplified product, the known volumetric ratio between the first and the second subsample and the known amount of the labelled second target analyte added in step (1a).

12. The assay method according to any one of claims 10-11, wherein the assay method further comprises determining the absolute concentration of at least a third target analyte in the sample.

13. A kit for determining an absolute concentration of a target analyte in a sample comprising:
- a first binder comprising a first nucleic acid target label having a first oligonucleotide sequence;
- a second binder comprising a second nucleic acid target label having a second oligonucleotide sequence;
- a first nucleic acid coupling agent; and
- a labelled target analyte comprising a label wherein:
(i) the first and second binder specifically bind to the target analyte and the labelled target analyte; and
(ii) the first and second oligonucleotide sequence interacts with the nucleic acid coupling agent.

14. The kit according to claim 13, wherein the labelled target analyte is biotinylated target analyte.

15. The kit according to any one of claims 13-14, further comprising:
- a third binder comprising a third nucleic acid target label having a third oligonucleotide sequence;
- a fourth binder comprising a fourth nucleic acid target label having a fourth oligonucleotide sequence;
- a second nucleic acid coupling agent; and
- a labelled second target analyte comprising a label wherein:
(i) the third and fourth binder specifically bind to the second target analyte and to the labelled second target analyte; and
(ii) the third and fourth oligonucleotide sequence interacts with the second nucleic acid coupling agent.
